# EUROPEAN PATENT APPLICATION

(11) **EP 3 588 072 A1**
(43) Date of publication of application: **01.01.2020**
(21) Application number: 19182597.5
(22) Date of filing: 26.06.2019
(51) Int. Cl.: G01N 27/30, G01N 27/327

(54) **METHOD FOR MANUFACTURING A WORKING ELECTRODE OF AN ELECTROCHEMICAL SENSOR AND PRODUCT THEREOF**

(30) Priority: 29.06.2018 TW 107122596
(71) Applicant: Phoenix Silicon International Corp., Hsinchu 300 (TW)
(72) Inventor: TSAI, Hsin-Chuan, 300 Taiwan (TW); LIAO, Hsueh-Chuan, 300 Taiwan (TW)
(74) Representative: Lermer, Christoph

(57) **Abstract**

A method for manufacturing a working electrode of electrochemical sensor comprises the steps of: providing a substrate; forming a defined pattern on the substrate; and disposing a plurality of conductive particles on the defined pattern.

## Description

### CROSS REFERENCE TO RELATED APPLICATION

This application claims the benefits of Taiwan application Serial No. 107122596, filed on June 29, 2018, the disclosures of which are incorporated by references herein in its entirety.

### TECHNICAL FIELD

The present invention relates to a method for manufacturing a working electrode of electrochemical sensor, and more particularly to a manufacturing method for improving the precision of existing three working electrodes of an electrochemical sensor. Semiconductor processes and nanotechnologies are applied in the method for manufacturing a working electrode of electrochemical sensor.

### BACKGROUND

Point-of-care testing (POCT) has been gaining more and more attention in resent years. The POCT can be rapid detection near to the patient in hospital bed or in clinic. Such rapid detection requires not only high sensitivity and high selectivity, but also small, inexpensive and easy to operate.

The most common example of the POCT is the blood glucose meter. Most of the blood glucose meters on the market use screen printing technology to make three-electrode disposable test strips. However, due to the poor precision of three-electrode test strips using screen printing technology, accurate blood glucose levels cannot be detected. The requirements for accuracy of the POCT are increasingly demanding.

### SUMMARY

An objective of the present invention is to solve the above-mentioned problems and to provide a method for manufacturing a working electrode of electrochemical sensor and product thereof, which can improve the precision of existing three working electrodes of electrochemical sensor. Semiconductor processes and nanotechnologies are applied in the method for manufacturing a working electrode of electrochemical sensor. The surface of the working electrode has a defined pattern to increase the detection region. The present invention can be used to immobilize enzymes, antibodies or nucleic acids on a working electrode for bio-sensing in various fields.

The present invention achieves the above-indicated objective by providing a method for manufacturing a working electrode of electrochemical sensor. The method comprises the steps of: providing a substrate; forming a defined pattern on the substrate; and disposing a plurality of conductive particles on the defined pattern.

In an embodiment of the present invention, a protective layer is formed on one side of the substrate.

In an embodiment of the present invention, a plurality of inverted triangular grooves with acute angles are formed on the substrate through the plurality of openings using anisotropic etching, or a plurality of semicircular grooves are formed on the substrate through the plurality of openings using isotropic etching.

In an embodiment of the present invention, the protective layer is removed. And the inverted triangular grooves with acute angles are formed into rounded grooves using isotropic etching, wherein a wavy pattern of rounded grooves is formed on the substrate and the wavy pattern is the defined pattern.

The present invention further discloses a working electrode of electrochemical sensor. The working electrode of electrochemical sensor comprises: a substrate; a defined pattern formed on the substrate; and a plurality of conductive particles disposed on the defined pattern.

In an embodiment of the present invention, the defined pattern includes a plurality of rounded grooves formed on the substrate.

In summary, the working electrode of the present invention has high precision and uses metal. The working electrode having nanometer particles of metal is fabricated into a chip. The surface of the detection region of the chip has a defined pattern. The defined pattern can increase the detection region to improve the accuracy of the detection. The working electrode of the present invention can be adhered to existing low-cost disposable test strips, and has a low-cost competitive advantage.

### BRIEF DESCRIPTION OF THE DRAWINGS

FIG. 1 is a flow chart of a method for manufacturing a working electrode of electrochemical sensor of the present invention.
FIG. 2 is a schematic view of the substrate having openings.
FIG. 3 is a schematic view of the substrate having a plurality of inverted triangular grooves.
FIG. 4 is a schematic view of the substrate with inverted triangular grooves and without a protective layer.
FIG. 5 is a schematic view of the substrate having a defined pattern.
FIG. 6 is a schematic view of the substrate having a conductive layer.
FIG. 7 is a schematic view of the substrate having the conductive layer and conductive particles.

### DETAILED DESCRIPTION

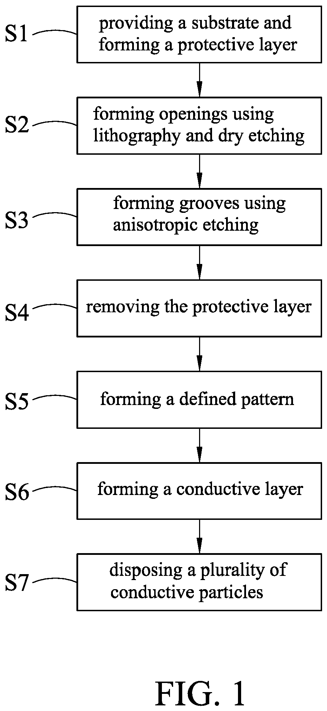

FIG. 1 is a flow chart of a method for manufacturing a working electrode of electrochemical sensor of the present invention. As shown in FIG. 1, a method for manufacturing a working electrode of electrochemical sensor comprises the following steps.

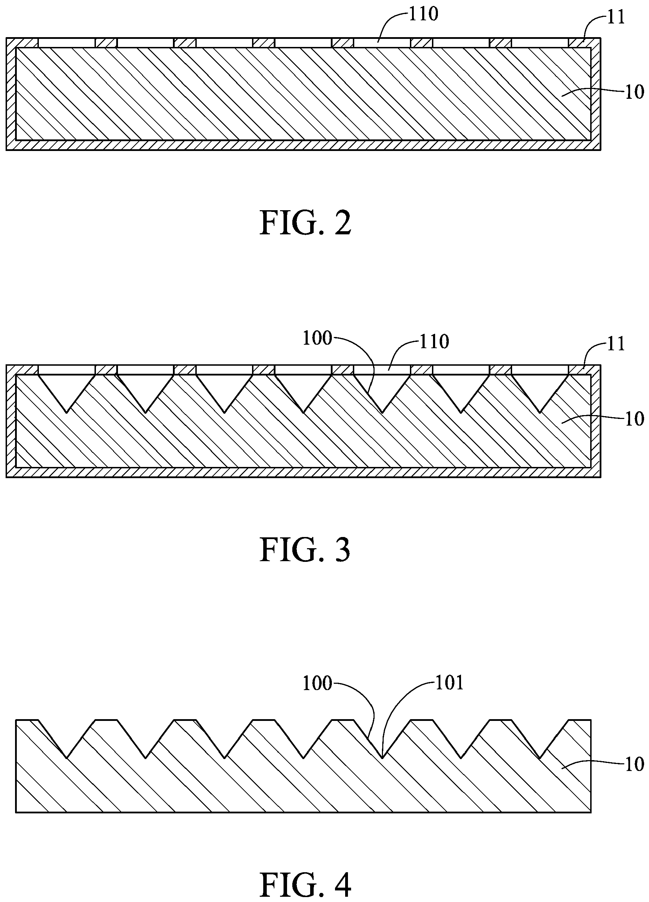

In step S1, providing a substrate, a substrate 10 is provided. A protective layer 11 is formed on one side of the substrate 10, as shown in FIG. 2. The substrate 10 is a silicon wafer having oxide or nitride with 1 to 100 ohm-cm resistivity.

In step S2, forming openings using lithography and dry etching, a plurality of openings 110 are formed on the protective layer 11 of the substrate 10 using lithography and dry etching.

In step S3, forming grooves using anisotropic etching, a plurality of inverted triangular grooves 100 are formed. The plurality of inverted triangular grooves 100 with acute angles are formed on the substrate 10 through the plurality of openings 110 using anisotropic etching, as shown in FIG. 3. An etching solution of the anisotropic etching is 10 to 45 wt% Potassium hydroxide KOH at a temperature between 40 and 90°C. Another etching solution can be 5 to 25 wt% tetramethylammonium hydroxide TMAH or TMAOH at a temperature between 40 and 90°C. Alternatively, a plurality of semicircular grooves (not shown) are formed on the substrate 10 through the plurality of openings 100 using isotropic etching.

In step S4, removing the protective layer, the protective layer 11 is removed. The substrate 10 has the plurality of inverted triangular grooves 100, and each of the grooves 100 has one acute angle 101 at the edge and bottom of the each groove 100, as shown in FIG. 4.

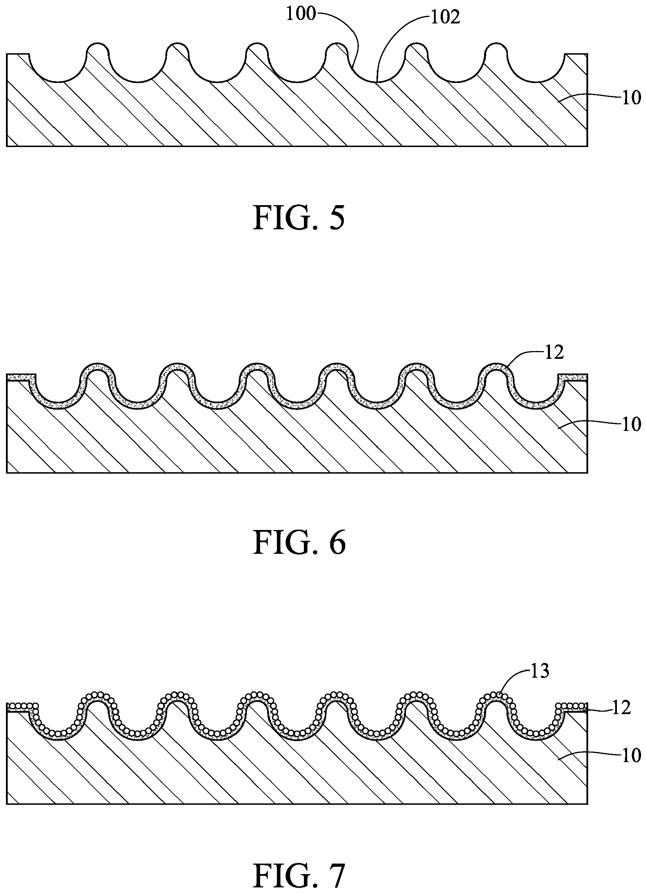

In step S5, forming a defined pattern, a defined pattern is formed using isotropic etching. The inverted triangular grooves 100 with acute angles 101 are formed into rounded grooves 102 using isotropic etching wherein a wavy pattern of rounded grooves 102 is formed on the substrate and the wavy pattern is the defined pattern, as shown in FIG. 5. An etching solution of the isotropic etching is one serving of 44-54 wt% hydrogen fluoride HF, three servings of 65-75 wt% nitric acid HNO₃ and eight servings of glacial acetic acid CH₃COOH at a temperature between 20 and 30°C.

In step S6, forming a conductive layer, a conductive layer 12 is formed on the defined pattern using sputtering or physical vapor deposition (PVD). The conductive layer 12 is formed on the wavy pattern of the substrate 10 using sputtering or physical vapor deposition, as shown in FIG. 6. The conductive layer 12 can be a chrome-copper (Cr-Cu) layer.

In step S7, disposing a plurality of conductive particles, a plurality of conductive particles are disposed on the defined pattern. A colloidal metal solution with conductive particles 13 and colloidal solution is disposed on the conductive layer 12, wherein the conductive particles 13 are formed on the conductive layer 12 after the colloidal solution dried, as shown in FIG. 7.

For example, the conductive particles 13 of the colloidal metal solution can generally be prepared by reducing Gold(III) chloride trihydrate solution with sodium citrate. The conductive particles 13 can be nanometer particles of metal having a size of from 1 to 10 nm. Where the nanometer particles of metal are nanometer particles of gold, the equation is:

HAuCl₄ + Na₃C₆H₅O₇ → nano Au

The present invention further discloses a working electrode of electrochemical sensor. The working electrode of electrochemical sensor comprises the substrate 10, the conductive layer 12 and the plurality of conductive particles 13, as shown in FIG. 7.

The plurality of inverted triangular grooves 100 with acute angles are formed on the substrate 10. The wavy pattern of rounded grooves 102 is formed on the substrate 10 and the wavy pattern is the defined pattern, as shown in FIG. 5.

The conductive layer 12 is formed on the inverted triangular grooves 100 of the substrate 10. The plurality of conductive particles 13 are formed on the conductive layer 12.

In summary, the working electrode of the present invention has high precision and uses metal. The working electrode having nanometer particles of metal is fabricated into a chip. The surface of the detection region of the chip has a defined pattern. The defined pattern can increase the detection region to improve the accuracy of the detection.

## Claims

1. A method for manufacturing a working electrode of electrochemical sensor, comprising the steps of:
providing a substrate;
forming a defined pattern on the substrate; and
disposing a plurality of conductive particles on the defined pattern.

2. The method for manufacturing a working electrode of electrochemical sensor as recited in claim 1, further comprising a step of forming a protective layer on one side of the substrate.

3. The method for manufacturing a working electrode of electrochemical sensor as recited in claim 2, further comprising a step of forming a plurality of openings on the protective layer using lithography and dry etching.

4. The method for manufacturing a working electrode of electrochemical sensor as recited in claim 3, further comprising a step of forming a plurality of inverted triangular grooves with acute angles on the substrate through the plurality of openings using anisotropic etching, or forming a plurality of semicircular grooves on the substrate through the plurality of openings using isotropic etching.

5. The method for manufacturing a working electrode of electrochemical sensor as recited in claim 4, further comprising the steps of:
removing the protective layer; and
forming the inverted triangular grooves with acute angles into rounded grooves using isotropic etching, wherein a wavy pattern of rounded grooves is formed on the substrate and the wavy pattern is the defined pattern.

6. The method for manufacturing a working electrode of electrochemical sensor as recited in claim 5, further comprising a step of forming a conductive layer on the defined pattern using sputtering or physical vapor deposition.

7. The method for manufacturing a working electrode of electrochemical sensor as recited in claim 6, further comprising a step of forming a colloidal metal solution with conductive particles and colloidal solution on the conductive layer, wherein the conductive particles are formed on the conductive layer after the colloidal solution dried.

8. A working electrode of electrochemical sensor, comprising:
a substrate;
a defined pattern, formed on the substrate; and
a plurality of conductive particles, disposed on the defined pattern.

9. The working electrode of electrochemical sensor as recited in claim 8, wherein the defined pattern includes a plurality of rounded grooves formed on the substrate.

10. The working electrode of electrochemical sensor as recited in claim 8, further comprising a conductive layer formed between the substrate and the plurality of conductive particles.
